# EUROPEAN PATENT SPECIFICATION

(11) **EP 2 510 915 B1**
(45) Date of publication and mention of the grant of the patent: **16.01.2019**
(21) Application number: 10835529.8
(22) Date of filing: 03.12.2010
(51) Int. Cl.: A61K 8/86, A61Q 1/00, A61Q 19/00, A61K 8/81, A61K 8/73, A61K 8/31, A61K 8/60, A61K 8/92

(54) **BASE FORMULATION FOR COSMETIC PRODUCTS**
BASISFORMULIERUNG FÜR KOSMETIKPRODUKTE
PRÉPARATION DE BASE POUR PRODUITS COSMÉTIQUES

(30) Priority: 11.12.2009 ES 200931147
(43) Date of publication of application: 17.10.2012
(73) Proprietor: Novejarque Conde, José Antonio, 46011 Valencia (ES)
(72) Inventor: Novejarque Conde, José Antonio, 46011 Valencia (ES)
(74) Representative: Isern Patentes y Marcas S.L.
(86) International application number: PCT/ES2010/070803
(87) International publication number: WO 2011/070207

(56) References cited:
- EP-A1- 0 770 378
- EP-A1- 1 905 411
- WO-A1-95/05796
- US-A- 4 664 914
- US-A1- 2007 259 012
- US-A1- 2008 233 064
- US-A1- 2009 035 244
- DATABASE GNPD [Online] MINTEL; 1 September 2009 (2009-09-01), Kosé: "Lipstick", XP002751434, Database accession no. 1199657
- "INTELLIGENT GEL TECHNOLOGY Versagel", , 24 April 2014 (2014-04-24), pages 1-1, XP055230828, USA Retrieved from the Internet: URL:http://www.penreco.com/images/spec-she ets/Intelligent_Gels.pdf [retrieved on 2015-11-24]

## Description

### Technical field

The present invention is framed within the field of cosmetics. Especially, it relates to a new base formulation for cosmetic products, characterized in that they present different degrees of texture and in that they are completely transparent.

### State of the art prior to the invention

In the last few years, a great variety of cosmetic products have been developed based on oils and gelling agents. In ES 2321495, for example, a gel-type product based on this type of compounds with improved structural and stability characteristics is presented.

One of the characteristics of the cosmetic products obtained from the formulation according to the present invention is their transparent or translucent appearance.

In literature, we can find patents such as, for example, ES 2125482, whose object of protection is aimed at a cosmetic product comprising a clear, single-phase composition. On the other hand, ES 2086932 describes a cosmetic composition for makeup, characterized by containing a transparent, titanium oxide and silicon oxide pigment.

The European patent EP 1905411 A1 discloses a colorless or colored transparent solid cosmetic comprising the components of: an amide-terminated polyamide resin, diisostearyl malate, polyglyceryl- 2 diisostearate, and a liquid oil.

The US patent application US 2007/0259012 refers to a solid, substantially clear cosmetic composition having a refractive index of between 1.4-1.6 comprising a partially crystalline polyolefin homopolymer or copolymer wax and a solvent system, wherein the refractive index of the solvent system is matched with the refractive index of the wax.

The object of this invention is to present a new base formulation for cosmetic products, characterized in that they present different degrees of texture and in that they are completely transparent. Generally, the viscosity of the final product will be relatively high and, in addition, it will exhibit a correct binding and homogenization of all the compounds present thereof, having, in turn, a practically insignificant water percentage.

### Description of the invention

The present invention relates to, therefore, a new base formulation for cosmetic products, characterized by comprising, in percentage by weight of the formulation and according to the INCI nomenclature:
a) from 0.2 to 10%, preferably from 0.5 to 6%, of at least one hydroxylated ester, consisting of PEG-20 glyceryl triisostearate, such as, for example , NIKKOL TGI-20, sold by Nikko Chemicals Co. Ltd.;
b) from 0.5 to 70%, preferably from 1 to 60%, of a mixture of at least one mineral oil and at least one copolymer derived from alkenes, consisting of an ethylene, propylene and styrene copolymer and a butylene, ethylene and styrene copolymer, as well as any combination thereof. This mixture may consist, for example, of VERSAGEL M-750, sold by Calumet Product Partners L.P.;
c) from 0.5 to 90%, preferably from 1 to 80%, of at least one hydrogenated polymer, consisting of polyalphaolefin, and more preferably, hydrogenated dodecene, such as, for example NEXBASE 2002, sold by Neste Oil;
d) from 0.5 to 35%, preferably from 1 to 25%, of a mixture of water and at least one glyceryl glucoside, such as, for example, GLYCOIN EXTREMIUM, sold by Jan Dekker Int.

The combination of the aforementioned compounds allows obtaining a formulation with optimal moisturizing and emollient characteristics, presenting a viscosity of 70.1 to 100,000 cps, where the minimum value corresponds to the viscosity of a conventional olive oil.

Preferably, the previous formulation may also comprise, in the percentage by weight of the formulation and according to the INCI nomenclature, at least one additional ingredient selected from a group consisting of:
e) a polysaccharide in a percentage of 0.05 to 8%, preferably from 0.1 to 3%, preferably selected between Chitosan and Beta-glucan, and any combination thereof, such as, for example, KIOSMETINE CG 125, sold by KitoZyme SA;
f) a natural extract in a percentage from 0.5 to 10%, preferably from 1 to 5%, preferably selected between an extract of Jojoba seed oil(Simmonidisia chinensis) and an extract of the fruit ofOpuntia Ficus Indica, as well as any combination thereof, such as, for example, REPARAMI, sold by Alban Muller Group Int.; and
g) a light medicinal oil in a percentage from 0.5 to 95%, preferably from 1 to 90%, and said medicinal oil is preferably constituted by a mixture of paraffinic and naphthenic hydrocarbons and is free from aromatic hydrocarbons, such as, for example, the one sold by QUIMIDROGA S.A.;as well as any combination thereof.

This way, the compounds comprising the formulation may consist of synthetic, natural or ecologic compounds.

Likewise, the formulation according to the invention may comprise another series of compounds selected from the ones normally used in the manufacturing of composite products. These compounds may be preferably selected, in a non-limiting manner, from a group consisting of odoriferous (perfumes, fragrances or aromas), stabilizing, pH-regulating and antioxidant substances, biocides and conservatives, medicinal, soothing, anti-cellulite, anti-wrinkle and anti-dandruff substances, hair straighteners, detangling, reaffirming, anti-corrosive, chelating, anti-foam, solvent, thickening, gelling, emollient, emulsifying, anti-binding, surfactant (anionic, cationic, amphoteric or non-ionic), emulsifying, exfoliating, moisturizing, humectant, makeup-removing, pigment-removing, pearlizing, solvent, deodorizing, antiperspirant, tanning, colouring, opacifying, whitening and bleaching substances, as well as any combination thereof.

The aforementioned compounds may consist, for example, of vitamins, emollient esters, lanolins, UV filters or protectors (antioxidant, organic or inorganic), vegetable oils, medicinal, pharmaceutical or cosmetic mineral oils (paraffinic, naphthenic or aromatic), essential oils, biological products, galenic products, plant extracts (flower or fruit extracts, dry extracts, glycolic, hydroglycolic, oily, colourless and watersoluble extracts), amino acids, alcohols, glycerines and vegetable glycerines, olive oil derivatives, vegetable petrolatums, silicones, fatty acids, oligo-elements, proteins, peptides, liposomes, active organic oils, natural active ingredients from vegetable, sea or synthetic origin, Aloe Vera and lipids, as well as any combination thereof.

Likewise, the object of this invention is a cosmetic product that comprises the aforementioned formulation. This product will be characterized by being hypoallergenic and non-irritating, as well as by presenting a transparent or translucent appearance, depending on the raw materials used in its manufacturing. Regarding the viscosity values of this cosmetic product, they are comprised between 70.1 y 100.000 cps.

According to another embodiment of the invention, the final cosmetic product may comprise from 0 to 7% by weight of water, without affecting its final properties. Said percentage may be pure deionized, osmotic and/or mineral water added, or it may come implicitly as a subcomponent of any of the compounds making up the formulation of the invention.

The cosmetic products according to the invention may be used in very diverse applications, such as, for example, the manufacturing of skin creams, emulsions, lotions, gels and oils; beauty masks (excluding chemical surface abrasion products for the skin); makeup (liquid, paste and powder); makeup powders, styling powder for after bathing and for body hygiene; beauty soap and deodorant soap; perfumes, eau de toilette and colognes; bath and shower products (salts, foams, oils and gels); depilatory products; deodorants and antiperspirants; hair products such as hair dyes and bleaches, products for moulding, straightening and setting hair, products that help maintaining the hairstyle, cleaning products (lotions, powders or shampoos), conditioning products (lotions or brilliantine hair sprays) and other hairstyling products; shaving products (soaps, foams, gels or lotions); face and eye makeup and makeup-removing products; lip products; mouth and teeth care products; nail makeup and care products; external intimate care products; sun care products; sunless tanning products; skin whitening or anti-wrinkle products.

Likewise, particularly, it may be used in the manufacturing of decorative-type cosmetic products such as, for example, lip makeup, eye shadow, blush, eyeliner, fluid makeup, powder makeup, mascara or nail polish.

Likewise, an additional object of this invention is the procedure to prepare the aforementioned formulation for cosmetic products. This procedure comprises, at least, the following steps:
a) mixing, while stirring continuously, the components of the formulation, excepting the mixture of at least one mineral oil and at least one copolymer derived from alkenes, until obtaining a complete and homogeneous mixture;
b) heating the aforementioned mixture to a temperature between 50 and 90°C;
c) adding the mixture of at least one mineral oil and at least one copolymer derived from alkenes to the aforementioned mixture, while stirring the mixture continuously during a period of time between 20 and 60 min;
d) cooling the aforementioned mixture to room temperature.

In a preferred embodiment of the invention, the aforementioned procedure will also comprise an additional packaging step for the formulation, in the desired dosed amounts.

Likewise, in a preferred embodiment in which the formulation of the invention comprises, additionally, at least one light medicinal oil, the same will be added to the manufacturing reactor after being weighted in an additional step prior to the addition of the remaining components.

### Preferred embodiment of the invention

We will now present, as an example and in a non-limiting manner, the description of a preferred embodiment of the present invention.

This way, a formulation was prepared according to the following percentages by weight:
NIKKOL TGI-20: 3%
KIOSMETINE CG. 125: 3%
VERSAGEL M-750: 50%
ECOLOGICAL ARGAN OIL: 30%
GLYCOIN™ EXTREMIUM: 4%
NEXBASE 2002: 10%

In the first place, the ecological argan oil was weighted and poured into the manufacturing reactor. Afterwards, with the reactor stirring at a speed of 100 to 500 rpm and at a temperature of 40 to 85°C, the remaining components were incorporated (NIKKOL TGI-20, KIOSMETINE CG. 125, GLYCOIN EXTREMIUM and NEXBASE 2002). Finally, VERSAGEL M-700 was slowly incorporated at a temperature of approximately 85°C in a homogeneous manner and by stirring continuously.

The final product obtained, aside from presenting a correct binding and homogenization of all the components of the formulation, allowed obtaining very satisfactory results, both for regenerating and nourishing the skin.

## Claims

1. Base formulation for cosmetic products, **characterized in that** it comprises, in percentage by weight of the formulation and according to the INCI nomenclature:
a) from 0.2 to 10% of at least one hydroxylated ester, wherein the hydroxylated ester consists of PEG-20 glyceryl triisosterate,
b) from 0.5 to 70% of a mixture of at least one mineral oil and at least one copolymer derived from alkenes, wherein said mixture consists of a mixture of mineral oil and at least one copolymer derived from alkenes selected from a group consisting of ethylene, propylene and styrene copolymer and a butylene, ethylene and styrene copolymer, as well as any combination thereof,
c) from 0.5 to 90% of at least one hydrogenated polymer, wherein the hydrogenated polymer consists of a polyalphaolefin,
d) from 0.5 to 35% of a mixture of water and at least one glyceryl glucoside.

2. Formulation, according to any claim 1, **characterized in that** it comprises, in percentage by weight of the formulation and according to the INCI nomenclature, at least one additional ingredient, selected from a group consisting of:
a) a polysaccharide in a percentage of 0.05 to 8%, preferably selected between Chitosan and Beta-glucan, as well as any combination thereof;
b) a natural extract in a percentage from 0.5 to 10%, preferably selected between an extract of Jojoba seed oil(Simmonidisia chinensis) and an extract of the fruit ofOpuntia ficus indica, as well as any combination thereof; and
c) a light medicinal oil in a percentage from 0.5 to 95%, as well as any combination of the previous ingredients.

3. Formulation, according to any one of the preceding claims, **characterized in that** it also comprises at least one additional compound selected from a group consisting of odoriferous, stabilizing, pH-regulating and antioxidant substances, biocides and conservatives, medicinal, soothing, anti-cellulite, anti-wrinkle and anti-dandruff substances, hair straighteners, detangling, reaffirming, anti-corrosive, anti-foam, solvent, thickening, chelating, gelling, emollient, emulsifying, anti-binding, surfactant, emulsifying, exfoliating, moisturizing, humectant, makeup-removing, pigment-removing, solvent, deodorizing, antiperspirant, colouring, pearlizing, opacifying, tanning, whitening and bleaching substances, as well as any combination thereof.

4. Formulation according to claim 3, in which said compound is selected from a group consisting of vitamins, emollient esters, lanolins, UV protectors, vegetable oils, mineral oils for medicinal, pharmaceutical or cosmetic use, essential oils, biological products, galenic products, plant extracts, amino acids, alcohols, glycerines, olive oil derivatives, vegetable petrolatums, silicones, fatty acids, oligo elements, proteins, peptides, liposomes, active organic oils, natural active ingredients from vegetable, sea or synthetic origin, Aloe Vera and lipids, as well as any combination thereof.

5. Cosmetic product **characterized in that** it comprises a formulation according to any one of the claims 1 to 4.

6. Use of a cosmetic product, according to claim 5, for the manufacturing of at least one product selected from skin creams, emulsions, lotions, gels and oils; beauty masks excluding chemical surface abrasion products for the skin; makeup; makeup powders, styling powder for after bathing and for body hygiene; beauty soap and deodorant soap; perfumes, eau de toilette and colognes; bath and shower products; depilatory products; deodorants and antiperspirants; hair products such as hair dyes and bleaches, products for moulding, straightening and setting hair, products that help maintaining the hairstyle and hair washing or conditioning products; shaving products; face and eye makeup and makeup-removing products; lip products; mouth and teeth care products; nail makeup and care products; external intimate care products; sun care products; sunless tanning products; skin whitening or anti-wrinkle products.

7. Use of a cosmetic product according to claim 5, for the manufacturing of a decorative-type cosmetic product selected from a group consisting of lip makeup, eye shadow, blush, eyeliner, fluid makeup, powder makeup, mascara and nail polish, as well as any combination thereof.

8. Preparation procedure for a formulation according to any one of the preceding claims 1 to 4, **characterized by** comprising, at least, the following steps:
a) mixing, while stirring continuously, the components of the formulation, excepting the mixture of at least one mineral oil and at least one copolymer derived from alkenes until obtaining a complete and homogeneous mixture;
b) heating of the aforementioned mixture to a temperature between 50 and 90 °C;
c) adding the mixture of at least one mineral oil and at least on copolymer derived from alkenes to the aforementioned mixture, while stirring the mixture continuously during a period of time between 20 and 60 min;
d) cooling the aforementioned mixture to room temperature.

9. Procedure, according to claim 8, **characterized by** comprising an additional initial step to add at least one light medicinal oil.

## Patentansprüche

1. Basisformulierung für kosmetische Produkte, **dadurch gekennzeichnet, dass** sie in Gewichtsprozent der Formulierung und gemäß der INCI-Nomenklatur Folgendes umfasst:
a) 0,2 bis 10% mindestens eines hydroxylierten Esthers, wobei der hydroxylierte Esther aus PEG-20 Glyceryl Triisosterat besteht,
b) 0,5 bis 70% einer Mischung aus mindestens einem Mineralöl und mindestens einem von Alkenen abgeleiteten Copolymer, wobei die Mischung aus einer Mischung aus Mineralöl und mindestens einem von Alkenen abgeleiteten Copolymer besteht, ausgewählt aus einer Gruppe bestehend aus Ethylen-, Propylen- und Styrol-Copolymer und einem Butylen-, Ethylen- und Styrol-Copolymer sowie einer beliebigen Kombination derselben,
c) 0,5 bis 90% mindestens eines hydrierten Polymers, wobei das hydrierte Polymer aus einem Polyalphaolefin besteht,
d) 0,5 bis 35% einer Mischung aus Wasser und mindestens einem Glycerylglucosid.

2. Formulierung nach Anspruch 1, **dadurch gekennzeichnet, dass** sie in Gewichtsprozent der Formulierung und gemäß der INCI-Nomenklatur mindestens einen zusätzlichen Inhaltsstoff umfasst, ausgewählt aus einer Gruppe bestehend aus:
a) einem Polysaccharid in einem Anteil von 0,05 bis 8%, vorzugsweise ausgewählt aus Chitosan und Beta-Glucan, sowie jede Kombination davon;
b) einem natürlichen Extrakt in einem Anteil von 0,5 bis 10%, vorzugsweise ausgewählt zwischen einem Extrakt aus Jojobasamenöl (Simmonidisia chinensis) und einem Extrakt aus der Frucht von Opuntia ficus indica, sowie jede Kombination davon; und
c) ein leichtes medizinisches Öl in einem Anteil von 0,5 bis 95%, sowie jede Kombination der vorhergehenden Inhaltsstoffe.

3. Formulierung nach einem der vorhergehenden Ansprüche, **dadurch gekennzeichnet, dass** sie auch mindestens eine zusätzliche Verbindung umfasst, die aus einer Gruppe ausgewählt ist, die aus duftenden, stabilisierenden, pH-regulierenden und antioxidativen Mitteln, Bioziden und Konservierungsmitteln, medizinischen, lindernden, Anti-Cellulite-, Anti-Falten- und Anti-Schuppen-Mitteln, Haarglättern, entwirrenden, straffenden, korrosionsschützenden, entschäumenden, lösenden, verdickenden, chelatbildenden, gelierenden, weichmachenden, emulgierenden, trennenden, oberflächenaktiven, emulgierenden, abschuppenden, feuchtigkeitsspendenden, feuchhaltenden, make-up-entfernenden, pigment-entfernenden, lösenden, deodorierenden, schweißhemmenden, färbenden, perlmutternden, trübenden, bräunenden, aufhellenden und bleichenden Mitteln besteht, sowie jede Kombination davon.

4. Formulierung nach Anspruch 3, wobei die Verbindung ausgewählt ist aus einer Gruppe bestehend aus Vitaminen, weichmachenden Esthern, Wollwachsen, UV-Schutzmitteln, Pflanzenölen, Mineralölen für medizinische, pharmazeutische oder kosmetische Zwecke, ätherischen Ölen, biologischen Produkten, galenischen Produkten, Pflanzenextrakten, Aminosäuren, Alkoholen, Glycerinen, Olivenölderivaten, pflanzlichen Petrolaten, Silikonen, Fettsäuren, Oligoelementen, Proteinen, Peptiden, Liposomen, aktiven organischen Ölen, natürlichen Wirkstoffen pflanzlichen, meeres- oder synthetischen Ursprungs, Aloe Vera und Lipiden, sowie jede Kombination davon.

5. Kosmetisches Produkt, **dadurch gekennzeichnet, dass** es eine Formulierung nach einem der Ansprüche 1 bis 4 umfasst.

6. Verwendung eines kosmetischen Produkts nach Anspruch 5 zur Herstellung von mindestens einem Produkt, ausgewählt aus Hautcremen, Emulsionen, Lotionen, Gelen und Ölen; Schönheitsmasken mit Ausnahme von chemischen oberflächenabrasiven Produkten für die Haut; Make-up; Make-up-Pulvern, Styling-Pulvern für nach dem Baden und für die Körperhygiene; Schönheitsseifen und Deodorantseifen; Parfümen, Eau de Toilette und Kölnischwasser; Bade- und Duschprodukten; Enthaarungsprodukten; Deodorants und Antitranspirants; Haarprodukten wie Haarfärbemittel und Bleichmittel, Produkten zum Formen, Glätten und Fixieren von Haaren, Produkten, die helfen, die Frisur aufrechtzuerhalten und Haarwasch- oder - konditionierungsprodukten; Rasierprodukten; Gesichts- und Augen-Make-up- und Make-up-Entfernungsprodukten; Lippenprodukten; Mund- und Zahnpflegeprodukten; Nagelmake-up- und Pflegeprodukten; externe Intimpflegeprodukten; Sonnenpflegeprodukten; sonnenfreie Bräunungsprodukten; Hautaufhellungs- oder Anti-Faltenprodukten.

7. Verwendung eines kosmetischen Produkts nach Anspruch 5 zur Herstellung eines dekorativen kosmetischen Produkts, ausgewählt aus einer Gruppe bestehend aus Lippen-Makeup, Lidschatten, Rouge, Eyeliner, Fluid-Makeup, Puder-Makeup, Mascara und Nagellack, sowie jede Kombination davon.

8. Herstellungsverfahren für eine Formulierung nach einem der vorhergehenden Ansprüche 1 bis 4, **dadurch gekennzeichnet, dass** es mindestens die folgenden Schritte umfasst:
a) Mischen der Bestandteile der Formulierung unter kontinuierlichem Rühren, mit Ausnahme der Mischung aus mindestens einem Mineralöl und mindestens einem von Alkenen abgeleiteten Copolymer, bis eine vollständige und homogene Mischung erhalten wird;
b) Erwärmen der vorgenannten Mischung auf eine Temperatur zwischen 50 und 90 °C;
c) Zugabe der Mischung aus mindestens einem Mineralöl und mindestens einem von Alkenen abgeleiteten Copolymer zu der vorgenannten Mischung unter kontinuierlichem Rühren der Mischung über einen Zeitraum zwischen 20 und 60 min;
d) Kühlen der vorgenannten Mischung auf Raumtemperatur.

9. Verfahren nach Anspruch 8 **dadurch gekennzeichnet, dass** es einen zusätzlichen anfänglichen Schritt zum Hinzufügen von mindestens einem leichten medizinischen Öl umfasst.

## Revendications

1. Formulation de base pour produits cosmétiques, **caractérisée en ce qu'**elle comprend, en pourcentage en poids de la formule et selon la nomenclature INCI :
a) de 0,2 à 10 % d'au moins un ester hydroxylé, dans laquelle l'ester hydroxylé consiste en triisostérate de PEG-20 glycéryle,
b) de 0,5 à 70 % d'un mélange d'au moins une huile minérale et d'au moins un copolymère issu d'alcènes, dans laquelle ledit mélange consiste en un mélange d'huile minérale et d'au moins un copolymère issu d'alcènes choisis dans un groupe consistant en copolymère d'éthylène, de propylène et de styrène et d'un copolymère de butylène, d'éthylène et de styrène, ainsi que n'importe quelle combinaison de ces derniers,
c) de 0,5 à 90 % d'au moins un polymère hydrogéné, dans laquelle le polymère hydrogéné consiste en une polyalphaoléfine,
d) de 0,5 à 35 % d'un mélange d'eau et d'au moins un glucoside de glycéryle.

2. Formulation, selon la revendication 1, **caractérisée en ce qu'**elle comprend, en pourcentage en poids de la formulation et selon la nomenclature INCI, au moins un ingrédient supplémentaire, choisi dans un groupe consistant en :
a) un polysaccharide dans un pourcentage de 0,05 à 8 %, de préférence choisi entre le Chitosan et le Bêta-glucane, ainsi que n'importe quelle combinaison de ces derniers ;
b) un extrait naturel dans un pourcentage de 0,5 à 10 %, de préférence choisi entre un extrait d'huile de graine de Jojoba (*Simmonidisia chinensis*) et un extrait du fruit de *Opuntia ficus indica,* ainsi que n'importe quelle combinaison de ces derniers ; et
c) une huile médicinale légère dans un pourcentage de 0,5 à 95 %, ainsi que n'importe quelle combinaison des ingrédients précédents.

3. Formulation, selon l'une quelconque des revendications précédentes, **caractérisée en ce qu'**elle comprend également au moins un composé supplémentaire choisi dans un groupe consistant en les substances odoriférantes, stabilisantes, régulatrices de pH et en les substances antioxydantes, les biocides et les conservateurs, les substances médicinales, apaisantes, anti-cellulite, anti-rides et anti-pelliculaires, des lisseurs capillaires, les substances démêlantes, réaffirmantes, anti-corrosives, anti-mousse, dissolvantes, épaississantes, chélatantes, gélifiantes, émollientes, émulsifiantes, anti-liantes, tensioactives, émulsifiantes, exfoliantes, hydratantes, humectantes, démaquillantes, dépigmentantes, dissolvantes, déodorantes, antitranspirantes, colorantes, nacrantes, opacifiantes, bronzantes, blanchissantes et décolorantes, ainsi que n'importe quelle combinaison de ces derniers.

4. Formulation selon la revendication 3, dans laquelle ledit composé est choisi dans un groupe consistant en les vitamines, les esters émollients, les lanolines, les protecteurs UV, les huiles végétales, les huiles minérales pour leur utilisation médicinale, pharmaceutique ou cosmétique, les huiles essentielles, les produits biologiques, les produits galéniques, les extraits de plante, les aminoacides, les alcools, les glycérines, les dérivés d'huile d'olive, les pétrolatums végétaux, les silicones, les acides gras, les oligoéléments, les protéines, les peptides, les liposomes, les huiles organiques actives, les ingrédients actifs naturels d'origine végétale, de mer ou synthétique, de l'Aloe Vera et des lipides, ainsi que n'importe quelle combinaison de ces derniers.

5. Produit cosmétique **caractérisé en ce qu'**il comprend une formulation selon l'une quelconque des revendications 1 à 4.

6. Utilisation d'un produit cosmétique, selon la revendication 5, pour la fabrication d'au moins un produit choisi parmi les crèmes pour la peau, les émulsions, les lotions, les gels et les huiles ; les masques de beauté à l'exception des produits chimiques d'abrasion superficielle pour la peau ; le maquillage ; les poudres de maquillage, la poudre coiffante post-bain et pour l'hygiène corporelle ; le savon de beauté et le savon déodorant ; les parfums, l'eau de toilette et les eaux de Cologne ; les produits de bain et de douche ; les produits dépilatoires ; les déodorants et les antitranspirants ; les produits capillaires tels que les teintures et les décolorants capillaires, les produits pour le modelage, le lissage et la fixation des cheveux, les produits qui permettent d'entretenir la coiffure et les produits de lavage ou les masques pour cheveux ; les produits de rasage ; le maquillage pour le visage et les yeux et les produits démaquillants ; les produits pour les lèvres ; les produits pour les soins buccaux et dentaires ; les produits pour le soin et le maquillage des ongles ; les produits pour les soins intimes externes ; les produits pour les soins solaires ; les produits autobronzants ; le blanchiment de la peau ou les produits anti-rides.

7. Utilisation d'un produit cosmétique selon la revendication 5, pour la fabrication d'un produit cosmétique de type décoratif choisi dans un groupe consistant en le maquillage pour les lèvres, les ombres à paupières, le blush, l'eyeliner, le maquillage fluide, le maquillage en poudre, le mascara et le vernis à ongles, ainsi que n'importe quelle combinaison de ces derniers.

8. Procédure de préparation pour une formulation selon l'une quelconque des revendications précédentes 1 à 4, **caractérisée en ce qu'**elle comprend, au moins, les étapes suivantes :
a) le mélange, tout en agitant continuellement, des composants de la formulation, à l'exception du mélange d'au moins une huile minérale et d'au moins un copolymère issu d'alcènes jusqu'à obtenir un mélange complet et homogène ;
b) le chauffage du mélange susmentionné à une température entre 50 et 90 °C ;
c) l'ajout du mélange d'au moins une huile minérale et d'au moins un copolymère issu d'alcènes au mélange susmentionné, tout en agitant le mélange continuellement pendant une période de temps entre 20 et 60 min ;
d) le refroidissement du mélange susmentionné à la température ambiante.

9. Procédure, selon la revendication 8, **caractérisée en ce qu'**elle comprend une étape initiale supplémentaire pour ajouter au moins une huile médicinale légère.
